# EUROPEAN PATENT SPECIFICATION

(11) **EP 2 758 522 B1**
(45) Date of publication and mention of the grant of the patent: **19.04.2017**
(21) Application number: 12761744.7
(22) Date of filing: 24.09.2012
(51) Int. Cl.: C12N 5/071, C07K 14/025, G01N 33/50

(54) **A HUMAN EPITHELIAL CELL LINE FOR 3D-MODELIZING OF CANCER AND TREATMENT THEREOF**
MENSCHLICHE EPITHELZELLLINIE ZUR 3D-MODELLIERUNG UND BEHANDLUNG VON KREBS
LIGNÉE CELLULAIRE ÉPITHÉLIALE HUMAINE POUR LA MODÉLISATION 3D DU CANCER ET TRAITEMENT ASSOCIÉ

(30) Priority: 23.09.2011 US 201161538602 P
(43) Date of publication of application: 30.07.2014
(73) Proprietor: Galderma Research & Development, 06410 Biot (FR); CNRS, 75794 Paris Cedex 16 (FR)
(72) Inventor: BURTY, Elodie, 69210 Sourcieux-les-Mines (FR); MAGNALDO, Thierry, 06100 Nice (FR); GACHE, Yannick, 06300 Nice (FR); AUBERT, Jérôme, 06130 Grasse (FR)
(74) Representative: Starck-Loudes, Anne-Caroline
(86) International application number: PCT/EP2012/068778
(87) International publication number: WO 2013/041726

(56) References cited:
- WO-A1-2009/015628
- WO-A1-2010/015618
- BRELLIER, F.; BERGOGLIO, V.; VALIN, A.; BARNAY, S.; CHEVALLIER-LAGENTE, 0.; VIELH, P.; SPATZ, A.; GORRY, P.; AVRIL, M.F.; MAGNALDO: "Heterozygous mutations in the tumor suppressor gene PATCHED provoke basal cell carcinoma-like features in human organotypic skin cultures", ONCOGENE, vol. 27, 2008, pages 6601-6606, XP2506351, cited in the application
- F. BRELLIER ET AL: "Ultraviolet responses of Gorlin syndrome primary skin cells", BRITISH JOURNAL OF DERMATOLOGY, vol. 159, no. 2, 1 August 2008 (2008-08-01), pages 445-452, XP055044573, ISSN: 0007-0963, DOI: 10.1111/j.1365-2133.2008.08650.x cited in the application
- BENJAMIN Z STANTON ET AL: "A small molecule that binds Hedgehog and blocks its signaling in human cells", NATURE CHEMICAL BIOLOGY, vol. 5, no. 3, 1 March 2009 (2009-03-01), pages 154-156, XP055017281, ISSN: 1552-4450, DOI: 10.1038/nchembio.142
- FRANCOISE BERNERD ET AL: "Reconstruction of DNA Repair-deficient Xeroderma Pigmentosum Skin In Vitro: A Model to Study Hypersensitivity to UV Light", PHOTOCHEMISTRY AND PHOTOBIOLOGY, vol. 81, 1 January 2005 (2005-01-01), pages 19-24, XP055044537, DOI: 10.1111/j.1751-1097.2005.tb01517.x cited in the application
- NAGANO K T ET AL: "Detecting tissue-specific alternative splicing and disease-associated aberrant splicing of the PTCH gene with exon junction microarrays", MEDLINE, 3 October 2005 (2005-10-03), XP003007188,

## Description

The present invention is in the domain of pharmacy and more specifically in skin cancer area, in particular Basal Cell Carcinoma (BCC). Inventors herein describe a cellular model targeting the Sonic Hedgehog/Patched (SHH/PTCH) pathway dysregulation or inappropriate activation as well as screening method using this cellular model to screen pharmacological compounds able to treat or prevent BCC lesions.
The Hedgehog pathway is normally active during embryonic development and plays a central role in cell differentiation and proliferation.
Inappropriate activation or dysregulation of the Hedgehog pathway is believed to play a critical role in the proliferation and survival of certain cancer cells, including in basal cell carcinoma and medulloblastoma.
Known pathway-activating mutations include those that impair the ability of PTCH, a transporter-like Hh receptor, to restrain Smoothened (SMO) activation of transcriptional targets via the GLI family of latent transcription factors.
Binding of Hh ligand to PTCH is functionally equivalent to genetic loss of PTCH, in that pathway activation by either requires activity of SMO, a seven pass transmembrane protein that binds to and is inactivated by the pathway antagonist, cyclopamine.
The implication of PATCHED pathway activation in several cancer conditions, most notably in BCCs, has motivated much effort to set up experimental systems to assess the inhibitory activity of small molecules.

The existing systems to measure activation or inhibition of the activated SHH/PTCH pathway, are based on cell lines from human or mouse origin, or else genetically engineered mice to develop susceptibility toward spontaneous or provoked BCC development. With respect to cells, they can schematically be classified in two categories destined to measure i) endogenous cellular events after treatment; these events include triggering of a differentiation process and modulation of gene expression, notably of those genes known as transcriptional targets of pathway activation; ii) cell lines engineered to report pathway activation/inhibition after transient or permanent introduction of reporter constructs made of responsive DNA driving a reporter gene; Cell lines developed so far are:
- Human normal primary keratinocytes and fibroblasts in reconstructed skin where expression of GL1 and GLI2 mRNA have been measured to demonstrate inhibition by the small Robotnikinin molecule of SHH/PTCH pathway activation by SHH.(Stanton et al. 2009)
- Healthy human primary keratinocytes from patients with nevoid basal cell carcinoma or Gorlin syndrome have been isolated to mimic the somatic loss of one PATCHED allele in sporadic BCC epidermal cells (Brellier et al., 2008a).

However, the above described cell lines have some disadvantages. Most of cell lines are not stable in the sense that after several passages the inserted genes expression decrease strongly or is shut down. Either those cell lines are not sufficiently robust to be efficient and sensitive to be used in a drug screening as a model. None of the cited prior art provides a system to allow a simple detection of activation in human epidermal keratinocytes.

On the other hand, animal models (mostly developed in the laboratory mouse) have been obtained by genetic engineering such as transgenesis of PTCH pathway activators (Gli1, Gli2, SHH, SMOM2) or abrogation of pathway inhibition by homologous recombination of the *PATCHED 1* gene. In this respect, it is, however, worth noting that mouse skin is i) very different from human skin (histology, DNA repair response, epidermal cell stemness) and ii) never develop BCC along life, even after massive chronic ultraviolet irradiations, or chemical treatment such as the two hits DMBA/TPA carcinogenesis protocol. Thus, results of carcinogenesis obtained in mice are not necessarily transposable to human cells.

In this context, there is a clear need for developing a human cell line easy to produce and to use, for developing efficient and relevant model sensitive enough for the screening or assessment of molecules libraries in a context respecting 3-dimensional architecture of human skin. Organotypic skin culture models composed of those cancer permissive cells may fulfil these specifications in the short term (2-3 weeks). In addition, humanized mice obtained after human skin regeneration using the said cell line(s) may serve to long term assessment of anticancer drug screening with highly relevant potential assessment.

The inventors have developed a new cell line providing strong advantages with the latter respect. An immortalized cell line of human keratinocytes with heterozygous mutation in the *PATCHED1* (PTCH 1) gene (KNBCCS6 E6/E7) is herein described. The cell line is stable overtime, particularly even after a high rate of passages in tissue culture.

In preferred embodiment of invention, the immortalized keratinocytes cell line according to the invention, express the PTCH1 mRNA and other members of the pathway necessary to appropriate activation and inhibition of the said SHH/PTCH pathway. Said cell line is immortalized by retroviral transduction of pLE6/E7SN. In addition, this cell line is produced in the absence of serum and without using feeder cells.

The description provides also a process for obtaining immortalized human keratinocytes as describe above, comprising the following steps of:
- isolating human primary keratinocytes from healthy individual with Nevoid basal cell carcinoma syndrome (see below for the impact of the said pathway in human genetic and sporadic disease both of which include BCC susceptibility as a common clinical trait),
- immortalizing human primary keratinocytes from NBCCS patients by retroviral transduction with pLE6/E7SN, and
- selecting cell line expression with a medium of selection.

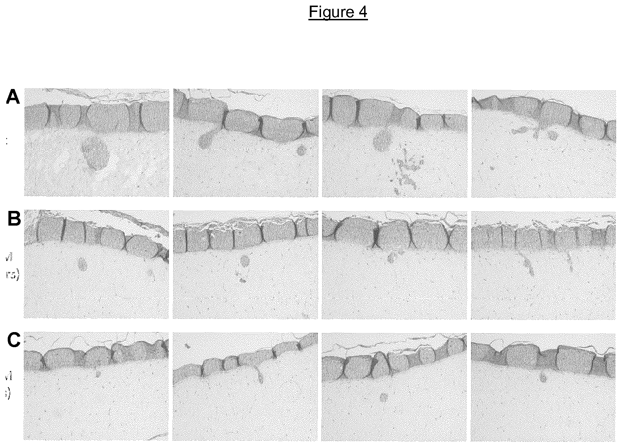

The description provides also a drug screening method, wherein said immortalized keratinocytes cell line as described above is used to screen. In a preferred aspect, the drug screening method comprises the following steps of:
a). bringing one samples of organotypic skin cultures reconstituted using immortalized cell line as herein described into contact with one or more of the test compounds,
b). measuring histology of skin equivalent to assess development of epidermal invasion in the dermal equivalent (figure 3) or other conservative method after stable labeling of the said cell line using GFP as described (Bergoglio 2007). By conservative method is meant that samples in vitro or in vivo, can be analysed in the real time without need of sample fixation and/or animal vector sacrifice (unless ethical rules should apply for sacrifice of animal vectors), and
c). selecting the compounds for which a modulation of epidermal invasion is measured in b) when compared with no drug mixture. In a preferred aspect, measurement of fluorescence emitted by GFP labeled cells in b) is used.

In a preferred aspect, the drug identified and or selected according to the drug screening method as described above is an anti-tumor drug.

The present description also provides an in vitro method for screening for candidate compounds for the preventive and/or curative treatment of cutaneous cancer and preferentially basal cell carcinoma, comprising the following steps of:
a. bringing one sample of immortalized cell line as described above into contact with one or more test compounds or a mixture of compounds;
b. measuring the invasive properties of the said cell line, and
c. selecting the compounds for which a modulation of invasion is measured in b) when compared with no drug mixture.

The description relates also to a drug obtainable with the drug screening method as described above.

### Detailed description

Basal cell carcinoma (BCC) of the skin is the commonest human cancer. BCCs derive from epidermal keratinocytes. The great majority of BCCs occurs on photo-exposed skin due to ultraviolet induced mutagenesis. The steadily rising incidence of BCCs in the last decades is attributed to increasing enthusiasm for recreational sun exposure. Although BCC rarely metastasize, they can result in local destruction and invasion of underlying tissues and consequently, life threatening complications. BCC are usually treated by local surgical excision, topical chemotherapy, photodynamic therapy, but, according to tumor size, location and frequency, there may be considerable aesthetic sequelae. Thus, drawbacks of current BCC treatments strongly support the need for pharmacological innovations that should specifically target the SONIC in so far as inappropriate and constitutive activation of this pathway is associated with the vast majority of BCC (see below). Furthermore, molecules that target the SHH pathway could also be of interest in the treatment of other non-BCC cancer conditions (or their stromal cells) (melanoma, pancreas, oesophagus, liver, prostate, lung, muscle, colon) where the SONIC HEDGEHOG/PATCHED (see below) has also been found inappropriately activated (Scales and de Sauvage, 2009).

### The SHH/ PATCHED pathway

The SHH/PTCH signalling pathway is essential during embryogenesis and development where it controls cell fate by modulating proliferation and differentiation. Animal models, notably the fruit fly drosophila melanogaster, have shown that at specific stages of development, some cells produce and emit a signal, the Hedgehog molecule (HH), which, in turn, is received by target cells. In vertebrates, the family of Hedgehog molecules is composed of Sonic Hedgehog, SHH, Desert Hedgehog, DHH, and Indian Hedgehog, IHH. Target cells (of these ligands) express PATCHED (PTCH), a putative twelve pass transmembrane protein acting as the receptor of HH molecules. When HH molecules are not expressed and/or not secreted at the vicinity of target cells, PTCH acts as a repressor of the pathway by inhibiting another transmembrane protein called SMOTHENED (SMO). SMO is a putative seven pass transmembrane protein related to G-protein coupled receptors. The inhibition of SMO by PTCH is relieved in the presence of HH molecules bound to PTCH. Derepression of SMO leads to activation of transcription factors of the GLI family (named GLI 1, 2 and 3) that activate (GLI1 and 2) or repress (GLI3), the transcription of their target genes. Interestingly, *PTCH1* is a transcriptional target of GLI1 and GLI2 factors.

The importance of the SHH/PTCH pathway is illustrated by severe diseases due to mutations affecting its integrity at different levels. Notably, in the human, heterozygous mutations in the *PTCH1* gene are responsible for the dominantly inherited genetic syndrome called nevoid basal cell carcinoma syndrome (NBCCS or Gorlin syndrome). NBCCS patients are highly prone to BCCs that generally (about 50 % cases) present with a loss of heterozygosity in the *PTCH1* locus. In Gorlin patients, more than 50% BCCs also bear mutation in the tumor suppressor gene *TP53,* suggesting some cooperation of the P53 and the SHH/PTCH pathways toward development of BCCs. Very interestingly, the two *PTCH1* alleles are also lost in most sporadic (general population) BCCs; in the latter case, again, the two *TP53* alleles are found mutated in 10 to 50% sporadic BCCs. 20-30 % sporadic BCCs are mutated in both *TP53* and *PTCH1* (Soufir et al., 2006). In both NBCSS and sporadic BCCs, inactivation of PTCH results in constitutive activation of the pathway with accumulation GLI1 and GLI2 mRNAs.

The implication of the SHH/PTCH pathway activation in several cancer conditions, most notably in BCCs, has motivated much effort to set up experimental systems to assess the inhibitory activity of small molecules.

The existing systems of activity measure are based on cell lines from human or mouse origin. Theses cells can schematically be classified in two categories destined to measure i) endogenous cellular events after treatment; these events include triggering of a differentiation process and modulation of gene expression, notably of those genes known as transcriptional targets of pathway activation; ii) cell lines engineered to report pathway activation/inhibition after transient or permanent introduction of reporter constructs made of responsive DNA driving a reporter gene. Cell lines developed so far in the prior art reveals that none of those system allows simple detection of activation in human epidermal keratinocytes in a context that reproduces 3-dimensional architecture of skin in the respect of dermo-epidermal interactions. As underlined above, models of genetically engineered mice are not necessarily relevant of human skin and cancer and hence assessment of relevant treatments to be applied thereof.

To provide a simple detection system, the inventors have worked to develop a human cell line in the respect of the following specifications (i.e. what we need for easy, efficient, relevant, sensitive assessment of molecules libraries):
- human cells,
- epidermal cells,
- growth in standard medium,
- needing no feeders,
- stably transformed by immortalizing antigen (E6/E7 from HPV 16)

To fulfil these specifications, the strategy was to use a human cell strain derived from normal human epidermis (Otto et al., 1999). Concerning the easiness of growth and the independency toward feeder cells, we decided to abrogate or at least to attenuate, the expression of the tumor suppressor gene *TP53.* Indeed, it is known for instance, that HaCat cells (that derive form an epidermal carcinoma with the two *TP53* allele mutated (Lehman et al., 1993)), are capable of growing in the absence of feeders cells. However, as explained below, use of these cells is not necessarily appropriate to measure pathway activation.

Previous work from the laboratory showed that P53 stabilisation after a single UVB irradiation is higher and prolonged in NBCCS compared to control keratinocytes (Brellier et al., 2008b). Also, report by Stecca and Ruiz-i-Altaba revealed mutual inhibition of Gli1 and P53 (Stecca and Ruiz i Altaba, 2009). Together with molecular epidemiology studies (showing mutations in both *TP53* and *PATCHED*, in 20-30 % BCCs), these results convergently indicate interaction between the P53 and the SHH/PTCH pathways (Soufir et al., 2006).

Thus, it is reasoned that abrogation or attenuation of the P53 pathway using E6-E7 oncogenic proteins of Human Papilloma Virus 16 (HPV16) would favor activation of the SHH/PTCH pathway in human epidermal keratinocytes. It must also be emphasized that, although they are generally mutated in *TP53,* keratinocytes from squamous cell carcinoma (SCC) are never mutated in *PTCH1* or at least do not show activation of the pathway (accumulation of target genes transcripts). This observation suggest that the SHH/PTCH pathway is not active in cells at the origin of SCCs (a subpopulation of epidermal stem cells). In spite of their easy growth in culture, SCC cells (such as HaCat cells) would thus not constitute appropriate recipient cells for reporter constructs. Preferably, it was decided to use human primary keratinocytes transformed with the E6-E7 oncogenic proteins.

Thus, the present description provides an immortalized cell line of human keratinocytes selected for specific growth properties and invasiveness in 3D culture conditions.
The immortalized keratinocyte cell line herein described expresses the PTCH protein. Said cell line is immortalized by retroviral transduction. The skilled in the art is familiar with retroviral transduction techniques. Any kind of retrovirus can be used such as Moloney murine leukemia virus (MoMLV), lentivirus, Eptein-Barr virus (EBV). MoMLV is preferred for high performance of infection in human primary keratinocytes (Bergoglio et al., 2007). The retroviral transduction of pLE6/E7SN is preferred in this context.

In addition, our previous observations have indicated that primary NBCCS primary fibroblasts isolated from healthy skin expressed a transcriptome resembling that characterized in fibroblasts associated to sporadic carcinomas (CAF) (Valin et al., 2009 ; Valin and Magnaldo, 2008). Together, these observations strongly support the idea of a strong contribution of dermal fibroblasts in carcinoma development in NBCCS patients. Thus, in the present description, skin permissive to epidermal invasion is reconstituted using the said immortalized keratinocytes cell line overvaying a dermal equivalent composed of NBCCS fibroblasts.

In addition, this cell line is produced in the absence of serum and without using feeder cells which provides a greater benefit in terms of feeding and growth time. Indeed, serum or feeder cells can incorporate or secrete substances which can by their presence interfere or modify the activity response. Cells are grown in a definite medium providing the advantage of growing cells in medium which does not interfere with activity response. A robust model with expected or calibrated response which avoids any interfering factors is thus herein described.

The description provides also a drug screening method, wherein said immortalized keratinocytes cell line as described above is used to screen. The description relates to an in vitro/in vivo screening method of PTCH pathway inhibitors for treating skin cancer and preferably BCC, comprising determining the capacity of said drug to inhibit or down regulate expression or biological activity of PTCH.

In a preferred aspect, the drug screening method comprises the following steps of:
a). bringing one samples of immortalized cell line as described above into contact with one or more of the test compounds;
b). measuring the invasiveness of the immortalized keratinocytes cell line as described above, and
c). selecting the compounds for which a modulation of invasiveness is measured in b) when compared with no drug mixture.

In the context of the description, cells are labelled with GFP, GFP derivatives, luciferase etc., reported with fluorescence or bioluminescence methods known by the skilled artisan. In a preferred aspect, the reporter/tracer gene is GFP.

The present description provides tools for selecting SHH/PTCH pathway modulators. Those modulators are activators or inhibitors.

In a preferred aspect, the drug identified and/or selected according to the drug screening method as described above is an anti-tumor drug. Inhibition efficacy of one or several drug candidates (isolated or in a mixture) preferably with increasing concentration, is assessed by histology or measure of reporter activity/tracer gene intensity of activity. The examples provide an illustration with a particular aspect in histological reporter system (figure 5).

In another aspect, the present description provides an in vivo tool for assessing keratinocyte invasiveness in a humanised animal model (such as mouse or mini-pig) having said KNBCCS6 E6/E7 immortalised cell line to follow activity of drug candidate kinetics The in situ assessment provides a considerable advantage with the GFP or derivatives fluorescence or any other reporter/tracer gene as the effect of drug candidate is eye visible and does not require further read out methods, nor does it require ending up of experiments including sample processing and vector animal sacrifice. This tool is also usable with reconstructed epidermis in vitro or skin regenerated in vivo.

The examples that follow illustrate the invention without limiting the scope thereof.

### EXAMPLES

### Materials and Methods

### Cell Culture

Human primary keratinocytes (named KNBCSS6 E6/E7) were isolated from a healthy non photo-exposed skin biopsy of NBCCS patient as described (Otto et al., 1999)

### Cell transformation and selection

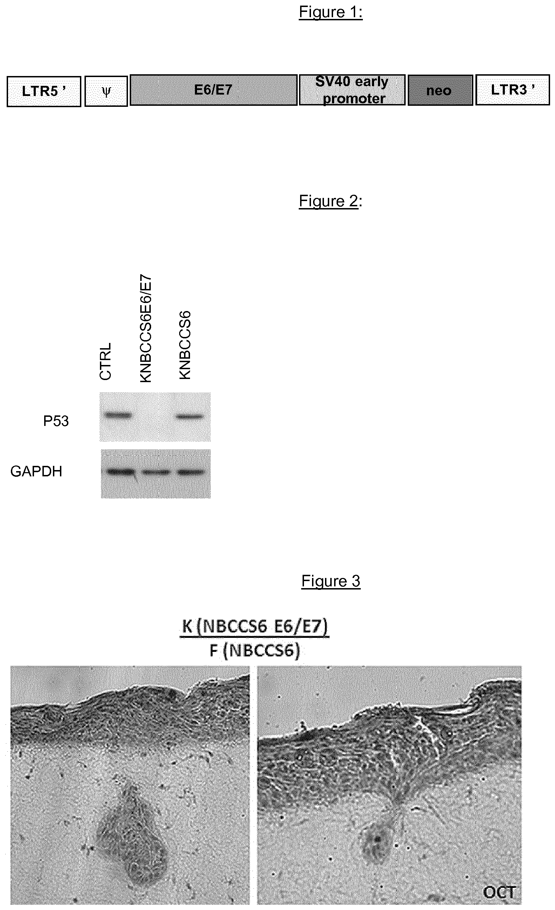

The NBCCS6 keratinocyte cell line was immortalized by retroviral transduction with pLE6/E7SN resulting in CTRL and NBCCS-*E6-E7* (figure 1) (Halbert et al., 1991, 1992). Cells were grown at 37 °C in a humidified atmosphere containing 5 % CO2, DMEM medium, 50 U/ml penicillin, 50 µg/ml streptomycin, 0.125 µg/ml amphotericin B, 2mM L-Glutamine, 1 mM Sodium pyruvate, 1x non essential amino acids.

G418 is an aminoglycoside antibiotic similar in structure to gentamicin B1, produced by Micromonospora rhodorangea. G418 blocks polypeptide synthesis by inhibiting the elongation step in both prokaryotic and eukaryotic cells. Resistance to G418 is conferred by the **Neomycin resistance gene (neo)** from Tn5 encoding an aminoglycoside 3'-phosphotransferase, APH 3' II.

Selection in mammalian cells is usually achieved in three to seven days with concentrations ranging from 200 to 1000 µg/ml (Arnaudeau-Bégard et al., 2003).

KNBCCS6 E6/E7 immortalized keratinocytes were grown at 37 °C in a humidified atmosphere containing 5 % CO2. Serum free medium (SFM Gibco ref: 10725-018), contained 0.1 mM CaCl₂, 2.10⁻⁴ ng/ml EGF, 50 U/ml penicillin, 50 µg/ml streptomycin, 0.125 µg/ml amphotericin B.

NBCC6 primary fibroblast cells were grown at 37 °C in a humidified atmosphere containing 5 % CO2, DMEM medium, 50 U/ml penicillin, 50 µg/ml streptomycin, 0.125 µg/ml amphotericin B, 2mM L-Glutamine, 1 mM Sodium pyruvate, 1x non essential amino acids.

### Small Molecules Modulators of the SHH/PTCH pathway

Purmorphamine (SMO agonist) and GDC-0449 (SMO antagonist) were diluted in DMSO at stock concentrations of 50 mM and 10 mM, respectively. To avoid side effects and toxicity, the final concentration of DMSO was fixed to 0.1 % DMSO.

### Transformation of the KNBCC6 keratinocyte cell line by the E6/E7 oncoproteins

Effective transformation of the KNBCCS6 cell line was assessed by analysing its growth properties and attenuation of expression of the P53 tumor suppressor protein. Figure 2 shows a western blot analysis of P53 in cell extracts prepared from preconfluent (about 80 %) primary epidermal keratinocytes (KNBCCS6), primary epidermal keratinocytes (KNBCCS6) after transformation using the E6E7 encoding retroviral vector (KNBCCS6 E6/E7). The western blot shows the drastic decrease in the amount of the P53 protein.

### Organotypic skin reconstruction using KNBCCS E6/E7 immortalized keratinocytes Overlaying a dermal equivalent containing autologous NBCCS primary fibroblasts:

Organotypic skin reconstruction using KNBCCS E6/E7 was performed using standard protocols as described in (Bernerd et al., 2001). In brief 10⁶ NBCCS6 primary fibroblasts cultured in standard culture medium as described above were embedded in a bovine collagen I lattice. After contraction (48hrs) lattices were fixed on plastic using a collagen glue, KNBCCS6 were then seeded (50 000 cells per lattice) within a stainless ring, left for 6 days in immersion; the day 6) before emersion (day 7), mounts were treated with appropriate concentration of SHH:PTCH pathway inhibitor with a final a 0.1 % DMSO concentration until the end of experiment. Control cultures were treated with 0.1 % DMSO. After 7 days of emersion at air-liquid interface, cultures were harvested for subsequent analyses including histology and assessment of tracer/reporter gene activity (cf. figure 3 and figure 4).

Figure 3 depicts the organotypic skin reconstruction showing invasive properties of KNBCC6 E6/E7 cell line over a dermal equivalent composed of autologous NBCCS6 primary fibroblasts

Figure 4 A, B & C depicts an organotypic skin reconstruction and effect of SHH/PTCH inhibitor and in particular figure 4 A, shows the same organotypic skin cultures system as in figure 3 composed of an epidermis developed from KNBCCS6 E6/ E7 immortalized keratinocyte overlaying a dermal equivalent containing autologous NBCCS6 primary fibroblasts. 4 fields are shown.

Figures 4B, 4C show the same as figure 4A, except that organotypic skin cultures were grown 24 hrs before being raised at air-liquid interface in the presence of 10 mM of GDC-0449 (B) or 10 mM (SANT1) SHH/PTCH pathway inhibitors. Note that the size and frequency of epidermal invasion were substantially decreased in the presence of either of both inhibitors.

### Conclusions

We have developed an immortalized human cell line of keratinocytes that respond to activation by an agonist (purmorphamine) and inhibition of the activation by an antagonist (GDC-0449, SANT1) of the SHH/PTCH signaling pathway.

The KNBCCS6 cell line does not need to be cultured in the presence of feeder cells. Theses cells can be expanded in a simple defined medium and they can grow easily due to immortalization. This tool is 3D organotypic culture system to evaluate the efficacy of antitumoral molecules specifically targeting inappropriate activation of the SHH/PTCH pathway occurring in BCC but also in numerous non BCC cancers including other cancer implicating inappropriate activation of the SHH/PTCH pathway. Assessment of inhibitor efficacy is applicable in the long term by skin regeration in vivo using animal vector including the mouse or the mini pig sytems.

### FIGURES

Figure 1: Schematic map of the LE6E7 SN proviral construct. E6E7, sequence of the human papilloma virus 16 encoding the E6 and E7 transforming proteins.
Figure 2: western blot analysis of the expression of the P53 protein in the indicated cells. GAPDH is a control of loading attesting that similar amount of protein is present in each lane.
Figure 3: Organotypic skin reconstruction showing invasive properties of KNBCC6 E6/E7 cell line over a dermal equivalent composed of autologous NBCCS6 primary fibroblasts
Figure 4: Organotypic skin reconstruction and effect of SHH/PTCH inhibitor

### REFERENCES

Arnaudeau-Bégard, C., Brellier, F., Chevallier-Lagente, O., Hoeijmakers, J.H., Bernerd, F., Sarasin, A., and Magnaldo, T. (2003). Genetic correction of DNA repair deficient/cancer prone xeroderma pigmentosum group C keratinocytes. Hum Gene Ther 14, 983-996.
Bergoglio, V., Larcher, F., Chevallier-Lagente, O., Bernheim, A., Danos, O., Sarasin, A., Rio, M.D., and Magnaldo, T. (2007). Safe selection of genetically manipulated human primary keratinocytes with very high growth potential using CD24. Mol Ther 15, 2186-2193.
Bernerd, F., Asselineau, D., Vioux, C., Chevallier-Lagente, O., Bouadjar, B., Sarasin, A., and Magnaldo, T. (2001). Clues to epidermal cancer proneness revealed by reconstruction of DNA repair-deficient xeroderma pigmentosum skin in vitro. Proc Natl Acad Sci U S A 98, 7817-7822.
Brellier, F., Bergoglio, V., Valin, A., Barnay, S., Chevallier-Lagente, O., Vielh, P., Spatz, A., Gorry, P., Avril, M.F., and Magnaldo, T. (2008a). Heterozygous mutations in the tumor suppressor gene PATCHED provoke basal cell carcinoma-like features in human organotypic skin cultures. Oncogene 27, 6601-6606.
Brellier, F., Valin, A., Chevallier-Lagente, O., Gorry, P., Avril, M.F., and Magnaldo, T. (2008b). Ultraviolet responses of Gorlin syndrome primary skin cells. Br J Dermatol 159, 445-452.
Halbert, C.L., Demers, G.W., and Galloway, D.A. (1991). The E7 gene of human papillomavirus type 16 is sufficient for immortalization of human epithelial cells. J Virol 65, 473-478.
Halbert, C.L., Demers, G.W., and Galloway, D.A. (1992). The E6 and E7 genes of human papillomavirus type 6 have weak immortalizing activity in human epithelial cells. J Virol 66, 2125-2134.
Lehman, T.A., Modali, R., Boukamp, P., Stanek, J., Bennett, W.P., Welsh, J.A., Metcalf, R.A., Stampfer, M.R., Fusenig, N., Rogan, E.M., et al. (1993). p53 mutations in human immortalized epithelial cell lines. Carcinogenesis 14, 833-839.
   Otto, A.I., Riou, L., Marionnet, C., Mori, T., Sarasin, A., and Magnaldo, T. (1999). Differential behaviors toward ultraviolet A and B radiation of fibroblasts and keratinocytes from normal and DNA-repair-deficient patients. Cancer Res 59, 1212-1218.
Scales, S.J., and de Sauvage, F.J. (2009). Mechanisms of Hedgehog pathway activation in cancer and implications for therapy. Trends Pharmacol Sci 30, 303-312.
Soufir, N., Gerard, B., Portela, M., Brice, A., Liboutet, M., Saiag, P., Descamps, V., Kerob, D., Wolkenstein, P., Gorin, I., et al. (2006). PTCH mutations and deletions in patients with typical nevoid basal cell carcinoma syndrome and in patients with a suspected genetic predisposition to basal cell carcinoma: a French study. Br J Cancer 95, 548-553.
Stecca, B., and Ruiz i Altaba, A. (2009). A GLI1-p53 inhibitory loop controls neural stem cell and tumour cell numbers. EMBO J 28, 663-676.
Valin, A., Barnay-Verdier, S., Robert, T., Ripoche, H., Brellier, F., Chevallier-Lagente, O., Avril, M.F., and Magnaldo, T. (2009). PTCH1 +/- dermal fibroblasts isolated from healthy skin of Gorlin syndrome patients exhibit features of carcinoma associated fibroblasts. PLoS One 4, e4818.
Valin, A., and Magnaldo, T. (2008). Method for determining a predisposition to basal cell carcinoma and for screening treatments therof. Brevet européen, depose le 05 Aout 2008, EP 08 3054486.

## Claims

1. An immortalized cell line of human keratinocytes derived from healthy individual having Nevoid Basal Cell Carcinoma Syndrome (NBCCS) with heterozygous mutation in the PATCHED 1 (PTCH 1) gene exhibiting stable expression overtime, wherein the cell line is immortalized by retroviral transduction with a retrovirus expressing the E6 and E7 genes of the human papilloma virus 16 (HPV16).

2. The cell line of immortalized keratinocytes according to claim 1, **characterized in that** it expresses PTCH 1 mRNA.

3. The cell line of immortalized keratinocytes according to claim 2, **characterized in that** it expresses the other members of the pathway Sonic Hedgehog/Patched (SHH/PTCH) necessary to appropriately inhibit and activate said pathway.

4. A process for obtaining an immortalized cell line of human keratinocytes as described in anyone of claims 1 to 3, comprising the following steps of:
- isolating human primary keratinocytes from a sample obtained from a healthy individual having Nevoid Basal Cell Carcinoma Syndrome (NBCCS),
- immortalizing the human primary keratinocytes from NBCCS patients by retroviral transduction with a retrovirus expressing the E6 and E7 genes of the human papilloma virus 16 (HPV16), and
- selecting cell line expression with a medium of selection.

5. The process of claim 4, wherein immortalized human keratinocytes are produced in the absence of serum or without using feeder cells.

6. An *in vitro* drug screening method, wherein a cell line of immortalized human keratinocytes according to anyone of claims 1 to 3 is used.

7. The drug screening method according to claim 6,comprising the following steps of:
a) bringing one sample of organotypic skin cultures reconstituted using a cell line of immortalized human keratinocytes according to anyone of claims 1 to 3 into contact with one or more test compounds or with a mixture of compounds,
b) measuring histology of skin equivalent to assess development of epidermal invasion in the dermal equivalent or other conservative method after stable labelling of the said cell line using GFP, and
c) selecting the compounds for which a modulation of epidermal invasion is measured in b) when compared to the invasion in the absence of any test compounds or mixture of compounds.

8. The drug screening method according to claim 7, wherein samples are analysed *in vitro.*

9. The drug screening method according to claim 6 or 7, wherein the identified drug is an antitumor drug.

10. An *in vitro* method for screening candidate compounds for use for the preventive and/or curative treatment of cutaneous cancer, comprising the following steps of:
a. bringing one sample of a cell line of immortalized human keratinocytes according to anyone of claims 1 to 3 into contact with one or more test compounds or with a mixture of compounds,
b. measuring the invasive properties of the said cell line, and
c. selecting the compounds for which a modulation of invasion is measured in b) when compared to invasion in the absence of any test compounds or mixture of compounds.

11. Organotypic skin cultures, reconstituted using immortalized cell line as described in anyone of claims 1 to 3, overlaying a dermal equivalent containing autologous NBCCS primary fibroblasts.

## Patentansprüche

1. Eine immortalisierte Zelllinie aus menschlichen Keratinozyten, erhalten von gesunden Individuen mit dem nevoiden Basalzellkarzinoma-Syndrom (NBCCS) mit heterogener Mutation im PATCHED1 (PTCH 1) Gen, welches eine stabile Expressions-Verlängerung aufweist, wobei die Zelllinie durch eine retrovirale Transduktion mit einem Retrovirus immortalisiert wurde, welches die E6- und E7-Gene des Humanen Papillomavirus 16 (HPV16) exprimiert.

2. Die Zelllinie aus menschlichen Keratinozyten nach Anspruch 1, dadurch charakterisiert, dass sie PTCH 1 mRNA exprimiert.

3. Die Zelllinie aus menschlichen Keratinozyten nach Anspruch 2, dadurch charakterisiert, dass sie die anderen Bestandteile des Sonic Hedgehog/Patched (SHH/PTCH) Signalweges exprimiert, die für eine entsprechende Inhibierung und Aktivierung des Signalweges benötigt werden.

4. Ein Verfahren zur Gewinnung einer immortalisierten Zelllinie aus menschlichen Keratinozyten wie beschrieben in einem der Ansprüche 1 bis 3, umfassend die folgenden Schritte:
- Isolieren von humanen primären Keratinozyten aus einer Probe erhalten von einem gesunden Individuum mit dem nevoiden Basalzellkarzinoma-Syndrom (NBCCS),
- Immortalisieren der humanen primären Keratinozyten von NBCCS-Patienten durch retrovirale Transduktion mit einem Retrovirus, welches die E6- und E7-Gene des Humanen Papillomavirus 16 (HPV16) exprimiert, und
- Selektionieren der Zelllinien-Expression mit einem Selektionsmedium.

5. Das Verfahren nach Anspruch 4, wobei die immortalisierten humanen Keratinozyten in Abwesenheit von Serum oder ohne die Verwendung von Feederzellen hergestellt werden.

6. Ein *in vitro* Wirkstoff-Durchmusterungsverfahren, wobei eine Zelllinie aus immortalisierten menschlichen Keratinozyten nach einem der Ansprüche 1 bis 3 verwendet wird.

7. Das Wirkstoff-Screening-Verfahren nach Anspruch 6, umfassend die folgenden Schritte:
a) In Kontakt bringen einer Probe von organotypischen Hautkulturen, die mit einer Zelllinie aus immortalisierten menschlichen Keratinozyten nach einem der Ansprüche 1 bis 3 rekonstituiert wurde, mit einer oder mehreren Testverbindungen oder mit einem Gemisch von Verbindungen,
b) Messen der Histologie von Hautäquivalenten, um die Ausbildung der epidermalen Invasion in das dermale Äquivalent zu bestimmen oder ein anderes konservatives Verfahren nach dem stabilen Markieren der Zelllinie mit GFP, und
c) Selektionieren der Verbindungen, für die eine Modulation der epidermalen Invasion im Vergleich zu einer Invasion in Abwesenheit von Testverbindungen oder einem Gemisch von Verbindungen in Schritt b) gemessen wird.

8. Das Wirkstoff-Durchmusterungsverfahren nach Anspruch 7, wobei die Proben *in vitro* analysiert werden.

9. Das Wirkstoff- Durchmusterungsverfahren nach Anspruch 6 oder 7, wobei der identifizierte Wirkstoff ein Antitumor-Wirkstoff ist.

10. Ein *in vitro* Verfahren zum Durchmustern von Wirkstoffkandidaten zur Verwendung zur präventiven und/oder kurativen Behandlung von Hautkrebs, umfassend die folgenden Schritte:
a. In Kontakt bringen einer Probe einer Zelllinie aus immortalisierten menschlichen Keratinozyten nach einem der Ansprüche 1 bis 3 mit einer oder mehreren Testverbindungen oder mit einem Gemisch von Verbindungen,
b. Messen der invasiven Eigenschaften der Zelllinie, und
c. Selektionieren der Verbindungen, für die eine Modulation der Invasion im Vergleich zu einer Invasion in Abwesenheit von Testverbindungen oder einem Gemisch von Verbindungen in Schritt b) gemessen wird.

11. Organotypische Hautkulturen, die mit einer Zelllinie wie beschrieben in einem der Ansprüche 1 bis 3 rekonstituiert wurde, überlagernd ein dermales Äquivalent enthaltend autologe primäre NBCCS Fibroblasten.

## Revendications

1. Lignée cellulaire immortalisée de kératinocytes humains dérivés d'un individu sain ayant un syndrome de carcinome basocellulaire naevoïde (NBCCS) avec une mutation hétérozygote dans le gène PATCHED1 (PTCH 1) présentant une expression stable dans le temps, dans laquelle la lignée cellulaire est immortalisée par transduction rétrovirale avec un rétrovirus exprimant les gènes E6 et E7 du virus du papillome humain 16 (HPV16).

2. Lignée cellulaire de kératinocytes immortalisés selon la revendication 1, **caractérisée en ce qu'**elle exprime un ARNm PTCH 1.

3. Lignée cellulaire de kératinocytes immortalisés selon la revendication 2, **caractérisée en ce qu'**elle exprime les autres membres de la voie Sonic hedgehog/Patched (SHH/PTCH) nécessaires pour inhiber ou activer de façon appropriée ladite voie.

4. Procédé d'obtention d'une lignée cellulaire immortalisée de kératinocytes humains telle que décrite dans l'une quelconque des revendications 1 à 3, comprenant les étapes suivantes :
- isolement de kératinocytes primaires humains à partir d'un échantillon obtenu à partir d'un individu sain ayant un syndrome de carcinome basocellulaire naevoïde (NBCCS),
- immortalisation des kératinocytes primaires humains provenant de patients NBCCS par transduction rétrovirale avec un rétrovirus exprimant les gènes E6 et E7 du virus du papillome humain 16 (HPV16), et
- sélection d'une expression d'une lignée cellulaire avec un milieu de sélection.

5. Procédé selon la revendication 4, dans lequel des kératinocytes humains immortalisés sont produits en l'absence de sérum ou sans utiliser de cellules nourricières.

6. Méthode *in vitro* de criblage de molécule thérapeutique, dans laquelle une lignée cellulaire de kératinocytes humains immortalisés selon l'une quelconque des revendications 1 à 3 est utilisée.

7. Méthode de criblage de molécule thérapeutique selon la revendication 6, comprenant les étapes suivantes :
a) mise en contact d'un échantillon de cultures de peau organotypiques reconstituées en utilisant une lignée cellulaire de kératinocytes humains immortalisés selon l'une quelconque des revendications 1 à 3 avec un ou plusieurs composés test ou avec un mélange de composés,
b) mesure de l'histologie d'un équivalent de peau pour évaluer un développement d'une invasion épidermique dans l'équivalent dermique ou une autre méthode conservatrice après un marquage stable de ladite lignée cellulaire en utilisant de la GFP, et
c) sélection de composés pour lesquels une modulation d'une invasion épidermique est mesurée à l'étape b) par comparaison avec l'invasion en l'absence de composés à tester ou d'un mélange de composés.

8. Méthode de criblage de molécule thérapeutique selon la revendication 7, dans laquelle les échantillons sont analysés *in vitro.*

9. Méthode de criblage de molécule thérapeutique selon la revendication 6 ou 7, dans laquelle la molécule thérapeutique identifiée est un médicament anti-tumoral.

10. Méthode *in vitro* de criblage de composés candidats pour une utilisation pour le traitement préventif et/ou curatif d'un cancer cutané, comprenant les étapes suivantes :
a. mise en contact d'un échantillon d'une lignée cellulaire de kératinocytes humains immortalisés selon l'une quelconque des revendications 1 à 3 avec un ou plusieurs composés test ou avec un mélange de composés,
b. mesure des propriétés invasives de ladite lignée cellulaire, et
c. sélection des composés pour lesquels une modulation d'une invasion est mesurée lors de l'étape b) par comparaison avec une invasion en l'absence de composés test ou d'un mélange de composés.

11. Cultures organotypiques de peau, reconstituées en utilisant une lignée cellulaire immortalisée telle que décrite dans l'une quelconque des revendication 1 à 3, recouvrant un équivalent dermique contenant des fibroblastes primaires NBCCS autologues.
